# EUROPEAN PATENT SPECIFICATION

(11) **EP 1 927 344 B1**
(45) Date of publication and mention of the grant of the patent: **09.06.2010**
(21) Application number: 07301608.1
(22) Date of filing: 30.11.2007
(51) Int. Cl.: A61K 8/41, A61Q 5/12

(54) **Compositions containing a quaternary ammonium polymer, a fatty quaternary agent and a nonionic surfactant**
Zusammensetzung mit einem quaternären Ammoniumpolymer, fettes quaternäres Mittel und nichtionisches Tensid
Compositions contenant un polymère d'ammonium quaternaire, un agent quaternaire gras et un tensio-actif non ionique

(30) Priority: 01.12.2006 US 872396 P
(43) Date of publication of application: 04.06.2008
(73) Proprietor: L'Oréal, 75008 Paris (FR)
(72) Inventor: ELLINGTON, Angela, FLOSSMORR, IL 60422 (US); HUNTER, Nikisha, CHICAGO, IL 60628 (US); PATTON, Carmen, SOUTH HOLLAND, IL 60473 (US)
(74) Representative: Martin-Charbonneau, Virginie

(56) References cited:
- EP-A- 1 142 557
- EP-A- 1 181 925
- WO-A-2004/041020
- WO-A-2006/050788

## Description

The present invention relates to an anhydrous composition based on a combination of at least one quaternary ammonium polymer, at least one fatty quaternary agent, and at least one nonionic surfactant.

The present invention also relates to a process for treating the hair, based on such an anhydrous composition that provides conditioning, moisturization, shine, and cosmetic feel, and helps reduce breakage.

Anhydrous hair care compositions such as pomades, for example, are usually made up of mineral oil and petrolatum and are used to enhance the feel and appearance of hair. With repeated use, however, these materials tend to leave an oily buildup and weigh hair down. Moreover, due to their high oil content, products of this nature tend to be difficult to rinse out of the hair.

A need therefore exists for a hair treatment composition and process that can reduce some of these undesirable effects and at the same time, can offer exceptional cosmetic feel and anti-frizz properties, plus restore moisturization and reduce breakage of the hair.

The present invention relates to a substantially anhydrous cosmetic composition containing:
(a) at least one quaternary ammonium polymer;
(b) at least one fatty quaternary agent;
(c) at least one nonionic surfactant; and
(d) optionally, at least one ceramide.
The present invention also relates to a process for treating a keratinous substrate involving contacting the substrate with the above-disclosed cosmetic composition.

Other than in the operating examples, or where otherwise indicated, all numbers expressing quantities of ingredients and/or reaction conditions are to be understood as being modified in all instances by the term "about".

As used herein, the expression "at least one" means one or more and thus includes individual components as well as mixtures/combinations.

The term "substantially anhydrous" means that the composition is either completely free of water or contains no more than 5% by weight, and more preferably no more than 1% by weight, based on the total weight of the composition.

"Cosmetically acceptable" means that the item in question is compatible with any keratin material. For example, "cosmetically acceptable medium" means a medium that is compatible with any keratinous substrate, and, in particular, human hair.

"Conditioning" as used herein means imparting to at least one keratinous fiber at least one property chosen from combability, manageability, moisture-retentivity, luster, shine, and softness. The state of conditioning is evaluated by measuring, and comparing, for example the ease of combability of the treated hair and of the untreated hair in terms of combing work (gm-in).

"Formed from," as used herein, means obtained from chemical reaction of, wherein "chemical reaction," includes spontaneous chemical reactions and induced chemical reactions. As used herein, the phrase "formed from", is open ended and does not limit the components of the composition to those listed, *e.g.,* as component (i) and component (ii). Furthermore, the phrase "formed from" does not limit the order of adding components to the composition or require that the listed components (*e.g.*, components (i) and (ii)) be added to the composition before any other components.

"Silicone compound," as used herein, includes, for example, silica, silanes, silazanes, siloxanes, and organosiloxanes; and refers to a compound comprising at least one silicon atom; wherein the silicone compound may be chosen from linear silicone compounds, branched silicone compounds, and cyclic silicone compounds; further wherein the silicone compound may optionally be substituted; and further wherein the silicone compound may optionally further comprise at least one heteroatom intercalated in the silicone chain, wherein the at least one heteroatom is different from the at least one silicon atom.

"Substituted," as used herein, means comprising at least one substituent. Non-limiting examples of substituents include atoms, such as oxygen atoms and nitrogen atoms, as well as functional groups, such as hydroxyl groups, ether groups, alkoxy groups, acyloxyalkyl groups, oxyalkylene groups, polyoxyalkylene groups, carboxylic acid groups, amine groups, acylamino groups, amide groups, halogen containing groups, ester groups, thiol groups, sulphonate groups, thiosulphate groups, siloxane groups, and polysiloxane groups.

"Keratinous substrate" as defined herein may be human keratinous fiber, and may be chosen from, for example, hair, eyelashes, and eyebrows, as well as the stratum corneum of the skin and nails.

"Polymers," as defined herein, include homopolymers and copolymers formed from at least two different types of monomers.

The cosmetic compositions and methods of the present invention can comprise, consist of, or consist essentially of, the essential elements and limitations of the invention described herein, as well as any additional or optional ingredients, components, or limitations described herein or otherwise useful in personal care compositions intended for topical application to hair and skin.

The present invention relates to a substantially anhydrous cosmetic composition for use in treating a keratinous substrate such as hair. The cosmetic composition is based on a mixture of at least one quaternary ammonium polymer, at least one fatty quaternary agent, at least one nonionic surfactant, and optionally, at least one ceramide. The present invention also relates to a process for treating the hair based on such an anhydrous composition involving contacting the substrate with the above-disclosed cosmetic composition.

Including water-soluble quaternary ammonium polymers and fatty quaternary agents in anhydrous compositions can be problematic in terms of formulating a stable composition. Given that many hair treatment and hair care compositions are emulsion aqueous based types of compositions, the incorporation of such ingredients into an anhydrous composition can be difficult.

It has been surprisingly found that the combination of a quaternary ammonium polymer, a fatty quaternary agent and a nonionic surfactant resulted in a stable and substantially anhydrous composition that is homogeneous and clear to substantially clear.

Accordingly, one aspect of the present invention is a care and/or treatment composition for keratinous substrates such as the hair which is able to address or overcome some or all of the aforementioned problems.

The present invention also relates to a method of conditioning, reducing breakage of and restoring moisture to the hair, such method comprising applying a substantially anhydrous composition comprised of at least one quaternary ammonium polymer, at least one fatty quaternary agent, at least one nonionic surfactant, and optionally, at least one ceramide to the hair, in an amount sufficient to condition, reduce breakage of and restore moisture to hair.

The present invention also relates to methods of treating, caring for and/or enhancing the appearance of keratinous substrates comprising applying compositions of the present invention to the keratinous substrates in an amount sufficient to treat, care for and/or enhance the appearance of the keratinous substrates.

It is to be understood that both the foregoing general description and the following detailed description are exemplary and explanatory only, and are not restrictive of the invention.

The composition of the present invention may be in any form suitable for use on keratinous substrates and can be, for example, leave-in conditioners, hair styling compositions, hair treatment compositions, scalp treatments, etc.

As defined herein, the composition of the present invention is considered stable when all ingredients are miscible and the composition has resisted any aesthetic change (such as two-phase separation, syneresis, etc.) over a specified period.

According to the present invention, the composition comprises at least one quaternary ammonium polymer. The at least one quaternary ammonium polymer is a polymer preferably comprising alkyl-substitued quaternary diamine monomers. For example, the quaternary ammonium polymer is preferably a polymer comprising repeating units of formula (a):

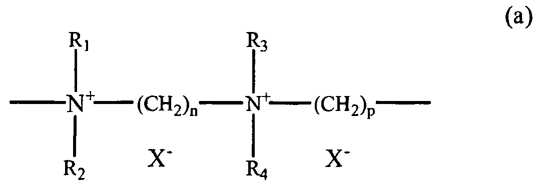

wherein:

R₁, R₂, R₃ and R₄, which may be identical or different, are each chosen from alkyl groups comprising from 1 to 4 carbon atoms and hydroxyalkyl groups comprising from 1 to 4 carbon atoms;

n and p, which may be identical or different, are each chosen from integers ranging from 2 to 20; and

X⁻ is an anion chosen for example from anions derived from inorganic acids and anions derived from organic acids.

Representative polymers of formula (a) include those in which R₁, R₂, R₃ and R₄ are chosen from methyl and ethyl groups and X⁻ is a halogen atom such as a halogen chosen from chlorine, iodine and bromine.

Further, representative polymers of formula (a) include polymers in which R₁, R₂, R₃ and R₄ are methyl groups and n=3, p=6 and X=Cl, such as those exemplified by formula (W): 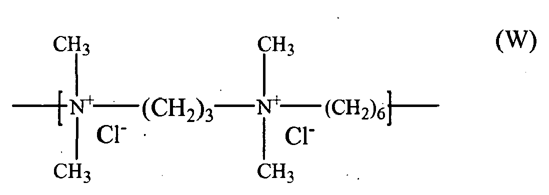 and of which the molecular weight, determined by gel-permeation chromatography, preferably ranges from 9500 to 9900.

Other polymers of formula (a) include those where R₁ and R₂ are methyl groups, R₃ and R₄ are ethyl groups and n=p=3 and X=Br, such as those exemplified by formula (U): 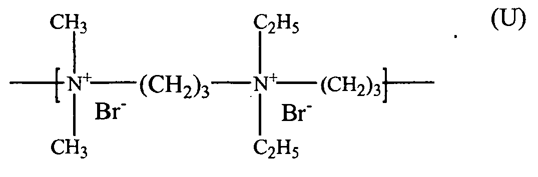 and of which the molecular weight, determined by gel-permeation chromatography, is preferably approximately 1200 .

The quaternary ammonium polymers of formula (a) can be prepared for example as described in French Patent 2,270,846, the entire disclosure of which is incorporated by reference herein.

Particularly preferred examples of the at least one quaternary ammonium polymer are polyquaternium-34 and hexadimethrine chloride, which is a polymer of N,N,N',N'-tetramethylhexamethylenediamine and trimethylene chloride and is disclosed in U.S. patent application publication no. 2002/0013972, the entire contents of which is hereby incorporated by reference.

Preferably, the at least one quaternary ammonium polymer is present in an amount of at least 0.001%, more preferably at least 0.1%, more preferably at least 0.2%, and more preferably at most 0.5% by weight with respect to the total weight of the composition. Preferred ranges are from 0.001% to 1.0% by weight of the total weight of the composition, more preferably from 0.1% to 1.0% of the total weight of the composition, more preferably from 0.1% to 0.5% of the total weight of the composition, and most preferably from 0.2% to 0.5%, including all ranges and subranges therebetween.

According to the present invention, the composition comprises at least one fatty quaternary agent.

The fatty quaternary agents useful in the present invention contain at least one quaternary nitrogen and a fatty chain having preferably from 6 to 22 carbon atoms. The anion of the quaternary agent can be a common ion such as chloride, ethosulfate, methosulfate, acetate, bromide, lactate, nitrate, phosphate, or tosylate and mixtures thereof. The long chain alkyl groups can include additional or replaced carbon or hydrogen atoms or ether linkages. Other substitutions on the quaternary nitrogen can be hydrogen, benzyl or short chain alkyl or hydroxyalkyl groups such as methyl, ethyl, hydroxymethyl or hydroxyethyl, hydroxypropyl or combinations thereof.

Examples of fatty quaternary agents include, but are not limited to: behentrimonium chloride, cocotrimonium chloride, cethethyldimonium bromide, dibehenyldimonium chloride, dihydrogenated tallow benzylmonium chloride, disoyadimonium chloride, ditallowdimonium chloride, hydroxycetyl hydroxyethyl dimonium chloride, hydroxyethyl behenamidopropyl dimonium chloride, hydroxyethyl cetyldimonium chloride, hydroxyethyl tallowdimonium chloride, myristalkonium chloride, PEG-2 oleamonium chloride, PEG-5 stearmonium chloride, PEG-15 cocoyl quaternium 4, PEG-2 stearalkonium 4, lauryltrimonium chloride; quaternium-16; quaternium-18, lauralkonium chloride, olealkonium chloride, cetylpyridinium chloride, polyquaternium-5, polyquaternium-6, polyquaternium-7, polyquaternium-10, polyquaternium-22, polyquaternium-37, polyquaternium-39, polyquaternium-47, cetyl trimonium chloride, dilauryldimonium chloride, cetalkonium chloride, dicetyldimonium chloride, soyatrimonium chloride, stearyl octyl dimonium methosulfate, behentrimonium methosulfate (18-MEA), stearalkonium chloride, and mixtures thereof. Other useful quaternary ammonium compounds are listed in the CTFA Cosmetic Ingredient Handbook, First Edition, on pages 41-42, incorporated herein by reference.

A preferred fatty quaternary agent is for example a mixture of behentrimonium methosulfate and C10-40 isoalkylamidopropylethyldimonium ethosulfate (Quaternium-33) such as the product sold under the name Incroquat® Behenyl 18-MEA, which is a mixture of behentrimonium methosulfate, C10-40 isoalkylamidopropylethyldimonium ethosulfate (Quaternium-33) and cetyl alcohol, commercially available from the company Croda, Inc.

In the composition of present invention, the at least one fatty quaternary agent is preferably present in an amount of from 0.001% to 2.0% by weight, preferably from 0.01% to 1.0% by weight, and more preferably from 0.1% to 0.5% by weight, based on the total weight of the composition.

According to the present invention, the composition comprises at least one nonionic surfactant.

In general, nonionic surfactants having a Hydrophilic-Lipophilic Balance (HLB) of 4 or more, preferably from 4 to 20, are contemplated for use by the present invention. Non-limiting examples of nonionic surfactants useful in the compositions of the present invention are disclosed in McCutcheon's "Detergents and Emulsifiers," North American Edition (1986), published by Allured Publishing Corporation; and McCutcheon's "Functional Materials," North American Edition (1992); both of which are incorporated by reference herein in their entirety.

Examples of nonionic surfactants useful herein include, but are not limited to, alkoxylated derivatives of the following compounds: fatty alcohols, alkyl phenols, fatty acids, fatty acid esters and fatty acid amides, wherein the fatty alkyl chain contains from 12 to 50 carbon atoms, preferably from 16 to 40 carbon atoms, more preferably from 16 to 24 carbon atoms, and having from 1 to 110 alkoxy groups. The alkoxy groups are selected from the group consisting of C2-C6 oxides and their mixtures, with ethylene oxide, propylene oxide, and their mixtures being the preferred alkoxides. The fatty alkyl chain may be linear, branched, saturated, or unsaturated. Of these alkoxylated non-ionic surfactants, the alkoxylated alcohols are preferred, and the ethoxylated alcohols and propoxylated alcohols are more preferred. The alkoxylated alcohols may be used alone or in mixtures thereof. The alkoxylated alcohols may also be used in mixtures with those alkoxylated materials disclosed hereinabove.

Non limitative representative examples of such ethoxylated fatty alcohols include laureth-3 (a lauryl ethoxylate having an average degree of ethoxylation of 3), laureth-23 (a lauryl ethoxylate having an average degree of ethoxylation of 23), ceteth-10 (a cetyl alcohol ethoxylate having an average degree of ethoxylation of 10) steareth-10 (a stearyl alcohol ethoxylate having an average degree of ethoxylation of 10), and steareth-2 (a stearyl alcohol ethoxylate having an average degree of ethoxylation of 2), steareth-100 (a stearyl alcohol ethoxylate having an average degree of ethoxylation of 100), beheneth-5 (a behenyl alcohol ethoxylate having an average degree of ethoxylation of 5), beheneth-10 (a behenyl alcohol ethoxylate having an average degree of ethoxylation of 10), and other derivatives and mixtures of the preceding.

The preferred ethoxylated fatty alcohols include oleth-3 (an oleyl ethoxylate having an average degree of ethoxylation of 3), oleth-5 (an oleyl ethoxylate having an average degree of ethoxylation of 5), and oleth-20 (an oleyl ethoxylate having an average degree of ethoxylation of 20), all commercially available from Croda, Inc

For example, such alkoxylated alcohols are available commercially under the name Brij® from Uniqema, Paterson, NJ. Typically, Brij® surfactants are the condensation products of fatty aliphatic alcohols with from 1 to 54 moles of ethylene oxide, the alkyl chain of the alcohol being typically a linear chain and having from 8 to 22 carbon atoms, for example, Brij 72 (i.e., steareth-2) and Brij 76 (i.e., steareth-10).

Also useful herein as nonionic surfactants are alkyl glycosides, which are the condensation products of long chain alcohols, e.g. C8-30 alcohols, with sugar or starch polymers. These compounds can be represented by the formula (S)ₙ-O-R wherein S is a sugar moiety such as glucose, fructose, mannose, galactose, and the like; n is an integer of from 1 to 1000, and R is a C8-30 alkyl group. Examples of long chain alcohols from which the alkyl group can be derived include decyl alcohol, cetyl alcohol, stearyl alcohol, lauryl alcohol, myristyl alcohol, oleyl alcohol, and the like.

Preferred examples of these surfactants are alkyl polyglucosides wherein S is a glucose moiety, R is a C8-20 alkyl group, and n is an integer of from 1 to 9. Commercially available examples of these surfactants include decyl polyglucoside (available as APG® 325 CS) and lauryl polyglucoside (available as APG® 600CS and 625 CS), all the above-identified polyglucosides APG® being available from the company Cognis, Ambler, Pa. Also useful herein are sucrose ester surfactants such as sucrose cocoate and sucrose laurate.

Other nonionic surfactants suitable for use in the present invention are glyceryl esters and polyglyceryl esters of fatty acids , including but not limited to, glyceryl monoesters, preferably glyceryl monoesters of C16-C22 saturated, unsaturated and branched chain fatty acids such as glyceryl oleate, glyceryl monostearate, glyceryl monoisostearate, glyceryl monopalmitate, glyceryl monobehenate, and mixtures thereof, and polyglyceryl esters of C16-C22 saturated, unsaturated and branched chain fatty acids, such as polyglyceryl-4 isostearate, polyglyceryl-3 oleate, polyglyceryl-2 sesquioleate, triglyceryl diisostearate, diglyceryl monooleate, tetraglyceryl monooleate, and mixtures thereof.

Also useful herein as nonionic surfactants are sorbitan esters. Preferable are sorbitan esters of C16-C22 saturated, unsaturated and branched chain fatty acids. Because of the manner in which they are typically manufactured, these sorbitan esters usually comprise mixtures of mono-, di-, tri-, etc. esters. Representative examples of suitable sorbitan esters include sorbitan monooleate (sold for example under the name SPAN® 80), sorbitan sesquioleate (sold for example under the name Arlacel® 83 by the company ICI Specialty Chemicals, Wilmington, Del.), sorbitan monoisostearate (sold for example under the name CRILL® 6 by the compaby Croda, Inc., Edison, N.J.), sorbitan stearates (sold for example under the name SPAN® 60), sorbitan trioleate (sold for example under the name SPAN® 85), sorbitan tristearate (sold for example under the name SPAN® 65), sorbitan dipalmitates (sold for example under the name SPAN® 40), and sorbitan isostearate. Sorbitan monoisostearate and sorbitan sesquioleate are particularly preferred emulsifiers for use in the present invention.

Also suitable for use herein as nonionic surfactants are alkoxylated derivatives of the following compounds: glyceryl esters, sorbitan esters, and alkyl polyglycosides, wherein the alkoxy groups is selected from the group consisting of C2-C6 oxides and their mixtures, with ethoxylated or propoxylated derivatives of these materials being the preferred. Nonlimiting examples of commercially available ethoxylated materials include the product sold by the company Uniqema under the name TWEEN® (ethoxylated sorbitan mono-, di- and/or tri-esters of C12 to C18 fatty acids with an average degree of ethoxylation of from 2 to 20).

.Preferred nonionic surfactants are those with an HLB of at least 4. HLB is understood to mean the balance between the size and strength of the hydrophilic group and the size and strength of the lipophilic group of the surfactant. Such derivatives can be polymers such as ethoxylates, propoxylates, polyglucosides, polyglycerins, polylactates, polyglycolates, polysorbates, and others that would be apparent to one of ordinary skill in the art. Such derivatives may also be mixed polymers of the above, such as ethoxylate/propoxylate species, where the total HLB is preferably greater than or equal to 4.

The nonionic surfactant is preferably present in the composition of the present invention in an amount ranging from 0.5% to 20% by weight, preferably from 1.0% to 10% by weight, and more preferably from 1.0% to 5.0% by weight, based on the total weight of the composition.

Optionally, ceramides may be used in the composition of the present invention for moisturizing the fiber and maintaining cuticle integrity. Ceramides are chosen from natural ceramides (available by extraction from natural sources), synthetic ceramides and pseudoceramides. Preferred ceramides are chosen from 2-oleamido-1,3-octanediol and the product sold under the name Ceramide II by the company Quest. Mixtures of ceramides may also be suitable, such as the product sold under the name Ceramides LS by Laboratories Serobiologiques.

Preferably, the ceramide may be present in the composition of the present invention in an amount ranging from 0.001% to 0.5% by weight of the total weight of the composition, more preferably from 0.01% to 0.5% of the total weight of the composition, and most preferably from 0.01% to 0.1%, including all ranges and subranges therebetween.

The composition of the present invention may contain further auxiliary ingredients.

Such auxiliary ingredients include conditioning agents, such as for example ester oils.

Suitable ester oils may be chosen from mono-, di-and tri- esters of glycerol. Preferred glyceride fatty esters are derived from carboxylic acids of carbon chain length ranging from 6 to 40 carbon atoms, preferably from 10 to 22 carbon atoms, and more preferably from 12 to 18 carbon atoms.

Synthetic ester oils which may be used include, but are not limited to, trimyristin, triolein, tristearin and glyceryl dilaurate. Vegetable derived glyceride fatty esters are particularly preferred, and specific examples of preferred materials as sources of glyceride fatty esters include peanut oil, sesame oil, avocado oil, coconut, cocoa butter, almond oil, safflower oil, corn oil, cotton seed oil, castor oil, hydrogenated castor oil, olive oil, jojoba oil, palm oil, soybean oil, wheat germ oil, linseed oil, and sunflower seed oil. Coconut oil, sunflower oil, castor oil and mixtures thereof are particularly preferred.

Particularly useful glyceryl ester oils herein include caprylic/capric triglyceride (for example the product Miglyol812, available from the company Degussa-Huls A G), triisostearin (for example the product SUN ESPOL G-318 available from the company Taiyo Kagaku), triolein (for example the product CITHROL GTO available from the company Croda, Inc.), trilinolein (for example the product EFADERMA-F available from the company Vevy or the product EFA-GLYCERIDES available from the company Brooks).

Additional examples of suitable esters include, for example, C12-15 alkyl benzoate (such as FINSOLV TN from Finetex, Elmwood Park, N.J.); octyl methoxy cinnamate (such as ESCALOL 557 from ISP, Wayne, N.J. (but in amounts less than 6% because of irritancy); isostearyl isostearate (such as (PRISORINE IS 2039 from Unichema, Chicago, Ill.); benzyl benzoate; 2,6-di-(ethylhexyl)naphthalate (such as Hallbrite TQ from the C.P. Hall Company, Bedford Park, Ill.); butyl octyl salicylate; glyceryl monostearate; n-dibutyl sebacate; isopropyl myristate; isopropyl palmitate; butyl stearate; cetyl lactate; isocetyl stearate; hexyl laurate; decyl oleate; isostearyl isostearate; ethyl hexyl maleate; sorbitan monoaurate; sorbitan monooleate; sorbitan sesquioleate; sorbitan trioleate; isopropyl palmitate; isopropyl stearate; stearyl stearate; diisopropyl adipate; diisopropyl sebacate; butyl myristate; isopropyl laurate; isotridecyl isononanoate; isostearyl neopentanoate; tridecyl neopentanoate; cetyl octanoate; cetyl ricinoleate; decyl isostearate; isodecyl oleate; isodecyl neopentanoate; isohexyl neopentanoate; tridecyl octanoate; alkyl benzoate; propyl myristate; propyl palmitate; tridecyl stearate; octyl palmitate and so on. Other suitable esters may also include esters such as tridecyl trimellilate, neopentyl glycol dicaprilate/dicaprate.

Also suitable may be synthetic or semi-synthetic glyceryl esters, e.g. fatty acid mono-, di-, and triglycerides which are natural fats or oils that have been modified, for example, acetylated castor oil, glyceryl stearate, glyceryl dioleate, glyceryl distearate, glyceryl trioctanoate, glyceryl linoleate, glyceryl myristate, glyceryl isostearate, PEG castor oils, PEG glyceryl oleates, PEG glyceryl stearates, PEG glyceryl tallowates, and so on.

Other conditioning agents may be chosen from natural, synthetic, saturated and unsaturated oils. Mention may be made of mineral oil, linear, branched and/or cyclic alkanes which may be volatile and, in particular, liquid paraffin, liquid petroleum jelly or hydrogenated polyisobutylene, isododecane or "Isopars", volatile isoparaffins.

Mention may also be made of aliphatic fatty liquid monoalcohols containing 6 to 40 carbon atoms, the hydrocarbon-based chain not comprising a substitution group. Monoalcohols according to the invention that may be mentioned include oleyl alcohol, decanol, octyldodecanol and linoleyl alcohol.

Mention may also be made of silicone oils such as for example polydimethylsiloxanes and polymethylphenylsiloxanes, optionally substituted with aliphatic and/or aromatic groups, which are optionally fluorinated, or with functional groups such as hydroxyl, thiol and/or amine groups, and volatile silicone oils, which are especially cyclic.

In particular, mention may be made of volatile and/or non-volatile, optionally branched silicone oils. The term "volatile oil" means an oil capable of evaporating from the skin or the lips in less than one hour, and especially having a vapor pressure, at room temperature and atmospheric pressure, ranging from 10⁻³ to 300 mmHg (0.13 Pa to 40,000 Pa).

As volatile silicone oils that may be used in the invention, mention may be made of linear or cyclic silicones containing from 2 to 7 silicon atoms, these silicones optionally comprising alkyl or alkoxy groups containing from 1 to 10 carbon atoms. Mention may be made in particular of octamethylcyclotetrasiloxane, decamethylcyclopentasiloxane, dodecamethylcyclohexasiloxane, heptamethylhexyltrisiloxane, heptamethyloctyltrisiloxane, octamethyltrisiloxane and decamethyltetrasiloxane, and mixtures thereof.

Among the non-volatile silicone oils that may be mentioned are non-volatile polydialkylsiloxanes, such as non-volatile polydimethylsiloxanes (PDMS); polydimethylsiloxanes comprising alkyl, alkoxy or phenyl groups, which are pendent or at the end of a silicone chain, these groups containing from 2 to 24 carbon atoms; phenyl silicones, for instance phenyl trimethicones, phenyl dimethicones, phenyl trimethyl-siloxy diphenylsiloxanes, diphenyl dimethicones, diphenyl methyldiphenyltrisiloxanes and polymethyl-phenylsiloxanes; polysiloxanes modified with fatty acids (especially of C₈-C₂₀), with fatty alcohols (especially of C₈-C₂₀) or with polyoxyalkylenes (especially polyoxy-ethylene and/or polyoxypropylene); amino polysiloxanes; polysiloxanes containing hydroxyl groups; fluoro polysiloxanes comprising a fluorinated group that is pendent or at the end of a silicone chain, containing from 1 to 12 carbon atoms, all or some of the hydrogen atoms of which are replaced with fluorine atoms; and mixtures thereof.

The at least one conditioning agent as defined above may be employed in the present composition in an amount of from greater than 0 to 95% by weight; greater than 0 to 50% by weight; greater than 0 to 25% by weight; greater than 0 to 15% by weight; greater than 0 to 10% by weight; greater than 0 to 5% by weight, based on the total weight of the composition.

Further additional ingredients that may be incorporated in the composition of the present invention include, but are not limited to alcohol esters, vitamin derivatives, colorants, and perfumes.

The compositions of the present invention may also contain adjuvants suitable for hair care. Suitable hair care adjuvants include, but are not limited to natural hair root nutrients, such as amino acids and sugars. Examples of suitable amino acids include arginine, cysteine, glutamine, glutamic acid, isoleucine, leucine, methionine, serine and valine, and/or precursors and derivatives thereof. The amino acids may be added singly, in mixtures, or in the form of peptides, e.g. di- and tripeptides. The amino acids may also be added in the form of a protein hydrolysate, such as a keratin or collagen hydrolysate. Suitable examples of sugars are glucose, dextrose and fructose. These may be added singly or in the form of, e.g. fruit extracts. A particularly preferred combination of natural hair root nutrients for inclusion in compositions of the invention is isoleucine and glucose. A particularly preferred amino acid nutrient is arginine.

Nonvolatile silicone compounds may also be employed as adjuvants in the compositions of the present invention such as, for example, a polydimethylsiloxane, polyalkyl siloxane, a polyaryl siloxane or a polyalkylaryl siloxane. The nonvolatile silicones are nonfunctional siloxanes or siloxane mixtures having a viscosity of about 10 to about 10, 000 cst , and most preferred viscosity about 10 to 500 cst at 25° C. A nonvolatile silicone compound is described as having a boiling point at atmospheric pressure of greater than about 250° C. A particularly preferred nonvolatile silicone compound is cyclopentasiloxane, commercially available from Dow Corning under the tradename DC 245.

Additional adjuvants include gelling agents, waxes, preservatives, suspending agents, volatile solvents, thickening agents, film formers, spreading agent, dispersants, antifoaming agents, wetting agents, UV-screening agents, antioxidants, perfumes, fillers, active agents, moisturizers, vitamins and their derivatives, biological materials, and derivatives of any of the foregoing.

The composition of the present invention possesses good conditioning and anti-frizz properties, reduces breakage of hair and provides shine to hair.

According to another embodiment of the present invention, there is also provided a process for treating a keratinous substrate, such as the hair, involving applying the above-disclosed composition onto said substrate. The process facilitates the styling and conditioning of substrates, such as hair, in a cosmetically appealing manner.

The following examples further describe and demonstrate embodiments within the scope of the present invention. The examples are given solely for the purpose of illustration and are not to be construed as limitations of the present invention, as many variations thereof are possible without departing from the scope of the invention. All exemplified amounts are concentrations by weight of the total composition, unless otherwise specified.

### EXAMPLES

The following compositions were prepared:

### Example 1

| | |
|---|---|
| Mineral oil and hydrogenated ethylene/propylene/styrene copolymer and hydrogenated butylene/ethylene/styrene copolymer (1) | 47.00 |
| C12-15 alkyl benzoate (2) | 2.00 |
| Oleth-5 (3) | 1.00 |
| Cetyl alcohol and behentrimonium methosulfate and quaternium 33 (4) | 0.01 |
| 2-oleamido-1,3-octadecanediol (5) | 0.001 |
| Hexadimethrine chloride (6) | 0.001 |
| Mineral oil (7) | Qsp 100 |

### Example 2

| | |
|---|---|
| Oleth-5 (3) | 5.00 |
| Cetyl alcohol and behentrimonium methosulfate and quaternium 33 (4) | 1.00 |
| 2-oleamido-1,3-octadecanediol (5) | 0.10 |
| Oleth-20 (8) | 5.00 |
| Hexadimethrine chloride (6) | 0.50 |
| Mineral oil (7) | Qsp 100 |

### Example 3

| | |
|---|---|
| Oleth-3 (9) | 5.00 |
| Cetyl alcohol and behentrimonium methosulfate and quaternium 33 (4) | 1.00 |
| 2-oleamido-1,3-octadecanediol (5) | 0.10 |
| Oleth-20 (8) | 5.00 |
| Hexadimethrine chloride (6) | 0.50 |
| Mineral oil (7) | Qsp 100 |

### Example 4

| | |
|---|---|
| Oleth-5 (3) | 5.00 |
| Cetyl alcohol and behentrimonium methosulfate and quaternium 33 (4) | 0.50 |
| 2-oleamido-1,3-octadecanediol (5) | 0.10 |
| Oleth-20 (8) | 5.00 |
| Hexadimethrine chloride (6) | 0.20 |
| Mineral oil (7) | Qsp 100 |

(1) sold under the name Versagel M1600 by the company Penreco
(2) sold under the name Finsolv TN by the company Finetex
(3) sold under the name Volpo 5 by the company Croda
(4) sold under the name Incroquat Behenyl 18 MEA by the company Croda
(5) sold under the name Mexanyl GZ by the company Chimex
(6) sold under the name Ionene G by the company Chimex
(7) sold under the name Mineral Oil by the company Penreco
(8) sold under the name brij 98V by the company Croda
(9) sold under the name Volpo 3 by the company Croda

## Claims

1. A substantially anhydrous cosmetic composition containing:
(a) at least one quaternary ammonium polymer;
(b) at least one fatty quaternary agent;
(c) at least one nonionic surfactant; and
(d) optionally, at least one ceramide;
said composition containing no more than 5% by weight of water, based on the total weight of the composition.

2. The composition of claim 1 wherein the at least one quaternary ammonium polymer comprises alkyl-substitued quaternary diamine monomers.

3. The composition of any preceding claim, wherein the at least one quaternary ammonium polymer is a polymer comprising repeating units of formula (a): 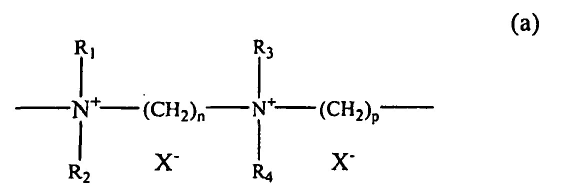 wherein:
R₁, R₂, R₃ and R₄, which may be identical or different, are each chosen from alkyl groups comprising from 1 to 4 carbon atoms and hydroxyalkyl groups comprising from 1 to 4 carbon atoms;
n and p, which may be identical or different, are each chosen from integers ranging from 2 to 20; and
X⁻ is an anion.

4. The composition of any preceding claim, wherein the at least one quaternary ammonium polymer is chosen from polyquaternium-34, hexadimethrine chloride, and mixtures thereof.

5. The composition of any preceding claim, wherein the at least one quaternary ammonium polymer is present in an amount of from 0.001% to 1.0% by weight of the total weight of the composition.

6. The composition of the preceding claim, wherein the at least one quaternary ammonium polymer is present in an amount of from 0.2% to 0.5% by weight of the total weight of the composition.

7. The composition of any preceding claim, wherein the at least one fatty quaternary agent contain at least one quaternary nitrogen and a fatty chain having from 6 to 22 carbon atoms.

8. The composition of any preceding claim, wherein the at least one fatty quaternary agent is chosen from behentrimonium chloride, cocotrimonium chloride, cethethyldimonium bromide, dibehenyldimonium chloride, dihydrogenated tallow benzylmonium chloride, disoyadimonium chloride, ditallowdimonium chloride, hydroxycetyl hydroxyethyl dimonium chloride, hydroxyethyl behenamidopropyl dimonium chloride, hydroxyethyl cetyldimonium chloride, hydroxyethyl tallowdimonium chloride, myristalkonium chloride, PEG-2 oleamonium chloride, PEG-5 stearmonium chloride, PEG-15 cocoyl quaternium 4, PEG-2 stearalkonium 4, lauryltrimonium chloride; quaternium-16; quaternium-18, lauralkonium chloride, olealkonium chloride, cetylpyridinium chloride, polyquaternium-5, polyquaternium-6, polyquaternium-7, polyquaternium-10, polyquaternium-22, polyquaternium-37, polyquaternium-39, polyquaternium-47, cetyl trimonium chloride, dilauryldimonium chloride, cetalkonium chloride, dicetyldimonium chloride, soyatrimonium chloride, stearyl octyl dimonium methosulfate, behentrimonium methosulfate, stearalkonium chloride, and mixtures thereof.

9. The composition of any preceding claim, wherein the at least one fatty quaternary agent is a mixture of behentrimonium methosulfate and C10-40 isoalkylamidopropylethyldimonium ethosulfate.

10. The composition of any preceding claim, wherein the at least one fatty quaternary agent is present in an amount of from 0.001% to 2.0% by weight of the total weight of the composition.

11. The composition of the preceding claim, wherein the at least one fatty quaternary agent is present in an amount of from 0.1% to 0.5% by weight of the total weight of the composition.

12. The composition of any preceding claim, wherein the at least one nonionic surfactant is chosen from alkoxylated derivatives of the following compounds: fatty alcohols, alkyl phenols, fatty acids, fatty acid esters and fatty acid amides, wherein the fatty alkyl chain contains from 12 to 50 carbon atoms, and having from 1 to 110 alkoxy groups.

13. The composition of the preceding claim, wherein the at least one nonionic surfactant is chosen from oleth-3, oleth-5, oleth-20, and mixtures thereof.

14. The composition of anyone of claims 1 to 11, wherein the at least one nonionic surfactant is chosen from:
- alkyl glycosides,
- glyceryl esters and polyglyceryl esters of fatty acids,
- sorbitan esters of C16-C22 saturated, unsaturated and branched chain fatty acids,
- alkoxylated derivatives of the following compounds: glyceryl esters, sorbitan esters, and alkyl polyglycosides,
- and mixtures thereof.

15. The composition of any preceding claim, wherein the at least one nonionic surfactant is present in an amount of from 0.5% to 20% by weight of the total weight of the composition.

16. The composition the preceding claim, wherein the at least one nonionic surfactant is present in an amount of from 1.0% to 5.0% by weight of the total weight of the composition.

17. The composition of any preceding claim, wherein at least one ceramide is chosen from natural ceramides, synthetic ceramides and pseudoceramides.

18. The composition of any preceding claim, wherein the at least one ceramide is present in an amount of from 0.001% to 0.5% by weight of the total weight of the composition.

19. A process for treating a keratinous substrate, involving contacting the substrate with a cosmetic composition according to anyone of claims 1 to 18.

20. The process of claim 19, wherein the keratinous substrate is hair.

21. A method of conditioning the hair, comprising applying to the hair a cosmetic composition according to anyone of claims 1 to 18.

## Patentansprüche

1. Im Wesentlichen wasserfreie kosmetische Zusammensetzung, die enthält:
(a) mindestens ein quartäres Ammoniumpolymer;
(b) mindestens eine quartäre Fettsubstanz;
(c) mindestens einen nichtionischen grenzflächenaktiven Stoff; und
(d) optional mindestens ein Ceramid;
wobei die Zusammensetzung, bezogen auf das Gesamtgewicht der Zusammensetzung, nicht mehr als 5 Gew.-% Wasser enthält.

2. Zusammensetzung nach Anspruch 1, wobei das zumindest eine quartäre Ammoniumpolymer alkylsubstituierte quartäre Diaminmonomere umfasst.

3. Zusammensetzung nach einem der vorhergehenden Ansprüche, wobei das zumindest eine quartäre Ammoniumpolymer ein Polymer ist, das Wiederholungseinheiten der Formel (a) enthält: 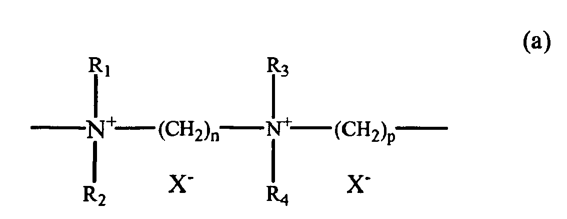 in der Formel:
die Gruppen R₁, R₂, R₃ und R₄, die identisch oder voneinander verschieden sein können, sind jede unter Alkylgruppen mit 1 bis 4 Kohlenstoffatomen und Hydroxyalkylgruppen mit 1 bis 4 Kohlenstoffatomen ausgewählt;
n und p, die identisch oder voneinander verschieden sein können, sind jeweils ganze Zahlen im Bereich von 2 bis 20; und
X- ist ein Anion.

4. Zusammensetzung nach einem der vorhergehenden Ansprüche, wobei das zumindest eine quartäre Ammoniumpolymer unter Polyquaternium-34, Hexadimethrine Chloride und deren Gemischen ausgewählt ist.

5. Zusammensetzung nach einem der vorhergehenden Ansprüche, wobei das zumindest eine quartäre Ammoniumpolymer in einem Mengenanteil von 0,001 bis 1,0 Gew.-% des Gesamtgewichts der Zusammensetzung enthalten ist.

6. Zusammensetzung nach dem vorhergehenden Anspruch, wobei das zumindest eine quartäre Ammoniumpolymer in einer Menge im Bereich von 0,2 bis 0,5 Gew.-% des Gesamtgewichts der Zusammensetzung vorliegt.

7. Zusammensetzung nach einem der vorhergehenden Ansprüche, wobei die zumindest eine quartäre Fettsubstanz mindestens ein quartäres Stickstoffatom und eine Fettkette mit 6 bis 22 Kohlenstoffatomen aufweist.

8. Zusammensetzung nach einem der vorhergehenden Ansprüche, wobei die zumindest eine quartäre Fettsubstanz unter Behentrimonium Chloride, Cocotrimonium Chloride, Cethethyldimonium Bromide, Dibehenyldimonium Chloride, Dihydrogenated Tallow Benzylmonium Chloride, Disoyadimonium Chloride, Ditallowdimonium Chloride, Hydroxycetyl Hydroxyethyl Dimonium Chloride, Hydroxyethyl Behenamidopropyl Dimonium Chloride, Hydroxyethyl Cetyldimonium Chloride, Hydroxyethyl Tallowdimonium Chloride, Myristalkonium Chloride, PEG-2 Oleamonium Chloride, PEG-5 Stearmonium Chloride, PEG-15 Cocoyl Quaternium 4, PEG-2 Stearalkonium 4, Lauryltrimonium Chloride; Quaternium-16; Quaternium-18, Lauralkonium Chloride, Olealkonium Chloride, Cetylpyridinium Chloride, Polyquaternium-5, Polyquaternium-6, Polyquaternium-7, Polyquaternium-10, Polyquaternium-22, Polyquaternium-37, Polyquaternium-39, Polyquaternium-47, Cetyl Trimonium Chloride, Dilauryldimonium Chloride, Cetalkonium Chloride, Dicetyldimonium Chloride, Soyatrimonium Chloride, Stearyl Octyl Dimonium Methosulfate, Behentrimonium Methosulfate, Stearalkonium Chloride und deren Gemischen ausgewählt ist.

9. Zusammensetzung nach einem der vorhergehenden Ansprüche, wobei die zumindest eine quartäre Fettsubstanz ein Gemisch von Behentrimonium Methosulfate und C 10-40 Isoalkylamidopropylethyldimonium Ethosulfate ist.

10. Zusammensetzung nach einem der vorhergehenden Ansprüche, wobei die zumindest eine quartäre Fettsubstanz in einer Menge im Bereich von 0,001 bis 2,0 Gew.-% des Gesamtgewichts der Zusammensetzung vorliegt.

11. Zusammensetzung nach dem vorhergehenden Anspruch, wobei die zumindest eine quartäre Fettsubstanz in einer Menge im Bereich von 0,1 bis 0,5 Gew.-% des Gesamtgewichts der Zusammensetzung vorliegt.

12. Zusammensetzung nach einem der vorhergehenden Ansprüche, wobei der zumindest eine nichtionische grenzflächenaktive Stoff unter den alkoxylierten Derivaten der folgenden Verbindungen ausgewählt ist: Fettalkoholen, Alkylphenolen, Fettsäuren, Fettsäurestern und Fettamiden, wobei die Alkyl-Fettkette 12 bis 50 Kohlenstoffatome umfasst und sie 1 bis 110 Alkoxygruppen aufweisen.

13. Zusammensetzung nach dem vorhergehenden Anspruch, wobei der zumindest eine nichtionische grenzflächenaktive Stoff unter Oleth-3, Oleth-5, Oleth-20 und deren Gemischen ausgewählt ist.

14. Zusammensetzung nach einem der Ansprüche 1 bis 11, wobei der zumindest eine nichtionische grenzflächenaktive Stoff ausgewählt ist unter:
- Alkylglycosiden,
- Gylcerylestern und Polyglycerylestern von Fettsäuren,
- Sorbitanestern von gesättigten, ungesättigten und verzweigten C₁₆₋₂₂-Fettsäuren,
- alkoxylierten Derivaten der folgenden Verbindungen: Glycerylestern, Sorbitanestern und Alkylpolyglycosiden,
- und deren Gemischen.

15. Zusammensetzung nach einem der vorhergehenden Ansprüche, wobei der zumindest eine nichtionische grenzflächenaktive Stoff in einer Menge im Bereich von 0,5 bis 20 Gew.-% des Gesamtgewichts der Zusammensetzung vorliegt.

16. Zusammensetzung nach dem vorhergehenden Anspruch, wobei der zumindest eine nichtionische grenzflächenaktive Stoff in einer Menge im Bereich von 1,0 bis 5,0 Gew.-% des Gesamtgewichts der Zusammensetzung vorliegt.

17. Zusammensetzung nach einem der vorhergehenden Ansprüche, wobei das zumindest eine Ceramid unter den natürlichen Ceramiden, synthetischen Ceramiden und Pseudoceramiden ausgewählt ist.

18. Zusammensetzung nach einem der vorhergehenden Ansprüche, wobei das zumindest eine Ceramid in einer Menge im Bereich von 0,001 bis 0,5 Gew.-% des Gesamtgewichts der Zusammensetzung vorliegt.

19. Verfahren zur Behandlung einer Keratinsubstanz, das umfasst, die Substanz mit einer kosmetischen Zusammensetzung nach einem der Ansprüche 1 bis 18 in Kontakt zu bringen.

20. Verfahren nach Anspruch 19, wobei es sich bei der Keratinsubstanz um das Haar handelt.

21. Verfahren zur Konditionierung der Haare, das umfasst, eine kosmetische Zusammensetzung nach einem der Ansprüche 1 bis 18 auf die Haare aufzubringen.

## Revendications

1. Composition cosmétique sensiblement anhydre, contenant :
a) au moins un polymère de type ammonium quaternaire,
b) au moins un agent quaternaire gras,
c) au moins un tensioactif non-ionique,
d) et en option, au moins un céramide,
laquelle composition ne contient pas plus de 5 % en poids d'eau,
par rapport au poids total de la composition.

2. Composition conforme à la revendication 1, dans laquelle ledit polymère de type ammonium quaternaire au nombre d'au moins un comporte des monomères de type diamine quaternisée à substituants alkyle.

3. Composition conforme à l'une des revendications précédentes, dans laquelle ledit polymère de type ammonium quaternaire au nombre d'au moins un est un polymère qui comporte des motifs répétitifs de formule (a) : 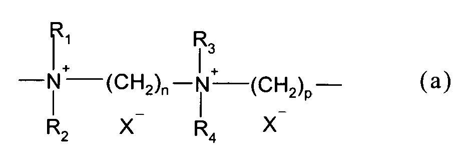 dans laquelle
R₁, R₂, R₃ et R₄, identiques ou différents, sont choisis chacun parmi les groupes alkyle comportant 1 à 4 atomes de carbone et les groupes hydroxyalkyle comportant 1 à 4 atomes de carbone ;
n et p sont des nombres entiers qui peuvent être identiques ou différents et qui ont chacun une valeur située dans l'intervalle allant de 2 à 20 ;
et X⁻ représente un anion.

4. Composition conforme à l'une des revendications précédentes, dans laquelle ledit polymère de type ammonium quaternaire au nombre d'au moins un est choisi parmi le polyquaternium-34, le chlorure d"hexadiméthrine et leurs mélanges.

5. Composition conforme à l'une des revendications précédentes, dans laquelle ledit polymère de type ammonium quaternaire au nombre d'au moins un est présent en une quantité de 0,001 à 1,0 % en poids rapporté au poids total de la composition.

6. Composition conforme à la revendication précédente, dans laquelle ledit polymère de type ammonium quaternaire au nombre d'au moins un est présent en une quantité de 0,2 à 0,5 % en poids rapporté au poids total de la composition.

7. Composition conforme à l'une des revendications précédentes, dans laquelle ledit agent quaternaire gras au nombre d'au moins un contient au moins un atome d'azote quaternaire et une chaîne grasse comportant de 6 à 22 atomes de carbone.

8. Composition conforme à l'une des revendications précédentes, dans laquelle ledit agent quaternaire gras au nombre d'au moins un est choisi parmi les composés suivants : chlorure de béhényl-triméthyl-ammonium, chlorure de (coco)-triméthyl-ammonium, bromure de cétyl-éthyl-diméthyl-ammonium, chlorure de dibéhényl-diméthyl-ammonium, chlorure de di(suif hydrogéné)-benzyl-méthyl-ammonium, chlorure de di(soja)-diméthyl-ammonium, chlorure de di(suif)-diméthyl-ammonium, chlorure d'hydroxycétyl-hydroxyéthyl-diméthyl-ammonium, chlorure d'hydroxyéthyl-béhénylamidopropyl-diméthyl-ammonium, chlorure d'hydroxyéthyl-cétyl-diméthyl-ammonium, chlorure d'hydroxyéthyl-(suif)-diméthyl-ammonium, chlorure de myrystalkonium, chlorure de (PEG-2)-oléyl-méthyl-ammonium, chlorure de (PEG-5)-stéaryl-méthyl-ammonium, (PEG-15)-cocoyl-quaternium-4, (PEG-2)-stéaryl-alkonium-4, chlorure de lauryl-triméthyl-ammonium, quaternium-16, quaternium-18, chlorure de lauryl-alkonium, chlorure d'oléyl-alkonium, chlorure de cétyl-pyridinium, polyquaternium-5, polyquaternium-6, polyquaternium-7, polyquaternium-10, polyquaternium-22, polyquaternium-37, polyquaternium-39, polyquaternium-47, chlorure de cétyl-triméthyl-ammonium, chlorure de dilauryl-diméthyl-ammonium, chlorure de cétyl-alkonium, chlorure de dicétyl-diméthyl-ammonium, chlorure de (soja)-triméthyl-ammonium, méthosulfate de stéaryl-octyl-diméthyl-ammonium, méthosulfate de béhényl-triméthyl-ammonium, et chlorure de stéaryl-alkonium, et les mélanges de ces composés.

9. Composition conforme à l'une des revendications précédentes, dans laquelle ledit agent quaternaire gras au nombre d'au moins un est un mélange de méthosulfate de béhényl-triméthyl-ammonium et d'éthosulfate d'(isoalkyl en C₁₀₋₄₀)-amidopropyl-éthyl-diméthyl-ammonium.

10. Composition conforme à l'une des revendications précédentes, dans laquelle ledit agent quaternaire gras au nombre d'au moins un est présent en une quantité de 0,001 à 2,0 % en poids rapporté au poids total de la composition.

11. Composition conforme à la revendication précédente, dans laquelle ledit agent quaternaire gras au nombre d'au moins un est présent en une quantité de 0,1 à 0,5 % en poids rapporté au poids total de la composition.

12. Composition conforme à l'une des revendications précédentes, dans laquelle ledit tensioactif non-ionique au nombre d'au moins un est choisi parmi les dérivés alcoxylés de composés des types suivants : alcools gras, alkyl-phénols, acides gras, esters d'acide gras et amides d'acide gras, dans lesquels la chaîne alkyle grasse comporte de 12 à 50 atomes de carbone et qui comportent de 1 à 110 motifs alkyl-oxy.

13. Composition conforme à la revendication précédente, dans laquelle ledit tensioactif non-ionique au nombre d'au moins un est choisi parmi les oleth-3, oleth-5, oleth-20 et leurs mélanges.

14. Composition conforme à l'une des revendications 1 à 11, dans laquelle ledit tensioactif non-ionique au nombre d'au moins un est choisi parmi :
- les alkyl-glycosides ;
- les esters de glycérol ou de polyglycérol et d'acides gras ;
- les esters de sorbitanne et d'acides gras en C₁₆₋₂₂ saturés ou insaturés et ramifiés ;
- les dérivés alcoxylés des esters de glycérol, des esters de sorbitanne et des alkyl-polyglycosides ;
- et les mélanges de tels composés.

15. Composition conforme à l'une des revendications précédentes, dans laquelle ledit tensioactif non-ionique au nombre d'au moins un est présent en une quantité de 0,5 à 20 % en poids rapporté au poids total de la composition.

16. Composition conforme à la revendication précédente, dans laquelle ledit tensioactif non-ionique au nombre d'au moins un est présent en une quantité de 1,0 à 5,0 % en poids rapporté au poids total de la composition.

17. Composition conforme à l'une des revendications précédentes, dans laquelle ledit céramide au nombre d'au moins un est choisi parmi les céramides naturels, les céramides synthétiques et les pseudo-céramides.

18. Composition conforme à l'une des revendications précédentes, dans laquelle ledit céramide au nombre d'au moins un est présent en une quantité de 0,001 à 0,5 % en poids rapporté au poids total de la composition.

19. Procédé de traitement d'un substrat kératinique, comportant le fait de mettre ce substrat en contact avec une composition cosmétique conforme à l'une des revendications 1 à 18.

20. Procédé conforme à la revendication 19, dans lequel le substrat kératinique est des cheveux.

21. Procédé de conditionnement des cheveux, comportant le fait d'appliquer sur les cheveux une composition cosmétique conforme à l'une des revendications 1 à 18.
